# EUROPEAN PATENT APPLICATION

(11) **EP 4 523 523 A1**
(43) Date of publication of application: **19.03.2025**
(21) Application number: 23803775.8
(22) Date of filing: 08.05.2023
(51) Int. Cl.: A01K 29/00, A61B 5/01, A61B 5/00, A01K 11/00, C09D 5/26

(54) **BODY TEMPERATURE DETECTION TAG**

(30) Priority: 09.05.2022 KR 20220056643
(71) Applicant: Lee, Sangseok, Ansan-si Gyeonggi-do 15541 (KR)
(72) Inventor: Lee, Sangseok, Ansan-si Gyeonggi-do 15541 (KR)
(74) Representative: Cleveland Scott York
(86) International application number: PCT/KR2023/006199
(87) International publication number: WO 2023/219352

(57) **Abstract**

The present disclosure is a body temperature detecting marker for checking body temperature of animals, comprising a display member; a coupling member configured to be capable of being separated from or coupled to the display member; and a color-changing member located between the display member and the coupling member and containing a thermochromic pigment that discolors to a different color or becomes transparent in a predetermined temperature range, wherein the display member is at least partially transparent or translucent so that a color change in the color-changing member is visible.

## Description

### FIELD OF THE INVENTION

The present disclosure relates to a body temperature detecting marker, and more specifically, to a body temperature detecting marker that can easily identify an animal with abnormal body temperature.

### BACKGROUND OF THE INVENTION

In most cases, infectious diseases in livestock are accompanied by fever. It is very important to prevent spread of disease by checking body temperature of disease-infected livestock and isolating them in early stages of a disease. However, in practice, it takes a lot of time and effort for a breeder to individually check for body temperature of numerous livestock.

For this purpose, the prior art has used a method of inserting a thermometer into a rectum of sick livestock to measure body temperature of sick livestock or a method of checking body temperature of the livestock through an image using an imaging camera.

However, the method of inserting a thermometer into the rectum of livestock may cause the livestock to be severely stressed during a process of the breeder approaching the sick livestock, measuring temperature, and inserting the thermometer into the rectum. This method of measuring body temperature increases stress on livestock, which can make livestock disease symptoms more severe. Furthermore, there is a risk of disease transmission if a breeder comes into contact with sick livestock and then touches another animal (livestock).

In addition, the method of measuring body temperature using an imaging camera can easily check for body temperature of a large number of livestock through images. However, the imaging camera is expensive, and after checking body temperature of the livestock, there is a hassle for the breeder to separately mark a sick animal.

### [Prior art]

### [Patent Document]

Korean Patent Application Publication No. 2019-0125882

### SUMMARY OF THE INVENTION

The purpose of the present disclosure is to provide a new method for easily and quickly checking livestock with abnormal body temperature among a large number of livestock by providing a body temperature detecting marker.

In an aspect of the present disclosure, provided herein is a body temperature detecting marker for checking body temperature of an animal, comprising: a display member; a coupling member configured to be capable of being separated from or coupled to the display member; and a color-changing member located between the display member and the coupling member and containing a thermochromic pigment that discolors to a different color or becomes transparent in a predetermined temperature range, wherein the display member is at least partially transparent or translucent so that a color change in the color-changing member is visible, the color-changing member further includes a cured polymer resin, and the color-changing member is applied on the display member, or on the display member and the coupling member.

In another aspect of the present disclosure, provided herein is a body temperature detecting marker for checking body temperature of an animal, comprising: a display member; a coupling member configured to be capable of being separated from or coupled to the display member; and a color-changing member located between the display member and the coupling member and containing a thermochromic pigment that discolors to a different color or becomes transparent in a predetermined temperature range, wherein the display member is at least partially transparent or translucent so that a color change in the color-changing member is visible, wherein the color-changing member includes a first color-changing member located to face one side of the an ear of the animal, and a second color-changing member located to face the other side of the ear of the animal.

In an embodiment, the predetermined temperature range may be 33 degrees Celsius to 43 degrees Celsius.

In an embodiment, the color-changing member may be located to face an epidermis of the animal.

In an embodiment, the color-changing member may be prepared by curing ink containing the thermochromic pigment and a solvent.

In an embodiment, the coupling member may include a first support plate, and a tip configured to protrude in one direction from one surface of the first support plate and penetrate a portion of the animal; and the display member may include a second support plate, and an accommodating part having a space into which a portion of the tip is inserted.

In an embodiment, the color-changing member may further contain a colored dye that does not change according to temperature.

In an embodiment, the body temperature detecting marker may further comprise a colored member that is interposed between the epidermis of the animal and the color-changing member, and does not discolor in a predetermined temperature range.

In an embodiment, the body temperature detecting marker may further comprise an elastic member between the color-changing member and the display member.

In an embodiment, the body temperature detecting marker may comprise a display member having a structure in which an outer periphery protrudes toward the ear of the animal.

### EFFECT

The body temperature detecting marker according to an embodiment of the present disclosure comprises a color-changing member, which is located between an at least partially transparent display member and a coupling member and contains a thermochromic pigment that discolors to a different color or becomes transparent in a predetermined temperature range, thereby easily checking body temperature change of an animal by naked eyes through a color change of the body temperature detecting marker.

In addition, the present disclosure does not use a thermometer to make contact as in the prior art, and thus does not aggravate a disease of a sick animal or artificially transmit the disease to another animal. Moreover, the present disclosure is economical, as it is less expensive than a conventional method of checking body temperature of the animal using a thermal imaging camera.

The body temperature detecting marker of the present disclosure does not require temperature measurement through separate contact unlike the method of using a thermometer in the prior art, and allows temperature of multiple animals to be checked by naked eyes at once, thereby easily checking body temperature of multiple animals.

The body temperature detecting marker of the present disclosure allows checking of body temperature of an animal by naked eye, isolates the sick animal at an early stage, slows down or block spread of the disease to other animals, which increases productivity of livestock farms and reduces damage caused by disease, thereby increasing economic profits. In addition, the present disclosure can speed up response to livestock infectious diseases such as foot-and-mouth disease and reduce damage caused by infectious diseases, thereby reducing disease management costs and reducing consumption of national budget.

### BRIEF DESCRIPTION OF THE DRAWINGS

FIG. 1 is a perspective view schematically showing an appearance of a body temperature detecting marker according to a first embodiment of the present disclosure.
FIG. 2 is a cross-sectional view schematically showing the appearance of the body temperature detecting marker according to the first embodiment of the present disclosure.
FIG. 3 is a perspective view schematically showing an appearance of a body temperature detecting marker according to a second embodiment of the present disclosure.
FIG. 4 is a cross-sectional view schematically showing the appearance of the body temperature detecting marker according to the second embodiment of the present disclosure.
FIG. 5 is a cross-sectional view schematically showing an appearance of a body temperature detecting marker according to a third embodiment of the present disclosure.
FIG. 6 is a cross-sectional view schematically showing an appearance of a body temperature detecting marker according to a fourth embodiment of the present disclosure.
FIG. 7 is a cross-sectional view schematically showing an appearance of a body temperature detecting marker according to a fifth embodiment of the present disclosure.
FIG. 8 is a cross-sectional view schematically showing an appearance of a body temperature detecting marker according to a sixth embodiment of the present disclosure.
FIG. 9 is a cross-sectional view schematically showing an appearance of a body temperature detecting marker according to a seventh embodiment of the present disclosure.
FIG. 10 is a cross-sectional view schematically showing an appearance of a body temperature detecting marker according to an eighth embodiment of the present disclosure.

### DESCRIPTION OF THE INVENTION

Advantages and features, and a method of achieving the same of the present disclosure will be apparent with reference to the embodiments described below in detail together with the accompanying drawings. However, the present disclosure is not limited to the embodiments disclosed below, but will be implemented in various forms, which are provided to ensure that the present disclosure is complete and to fully inform the scope of the invention to those skilled in the art to which the present disclosure pertains, and the present disclosure will be only defined by the scope of the claims.

The terms used in the present specification are used only to describe specific embodiments and are not used with the intention of limiting the present disclosure. For example, a component represented by a singular number should be understood as a concept containing multiple components unless the context explicitly means only a singular number. Also, in the specification of the present disclosure, terms such as 'include' or 'have' are intended to designate that there is a feature, number, step, operation, component, part, or combination thereof described in the specification, but the use of these terms does not exclude the possibility of the presence or addition of one or more other features, numbers, steps, operations, components, parts, or combinations thereof.

In addition, all terms used herein including technical or scientific terms, unless otherwise defined, have the same meaning as those generally understood by those skilled in the art to which the present disclosure pertains.

Terms such as those defined in commonly used dictionaries should be interpreted as having a meaning consistent with the context of the relevant technology and not be interpreted as being idealized or excessively formal unless explicitly defined in the specification of the present disclosure.

Hereinafter, embodiments of the present disclosure will be described in more detail with reference to the accompanying drawings. However, in the following description, if there is a concern that the gist of the present disclosure is unnecessarily blurred, a detailed description related to a widely known function or configuration will be omitted.

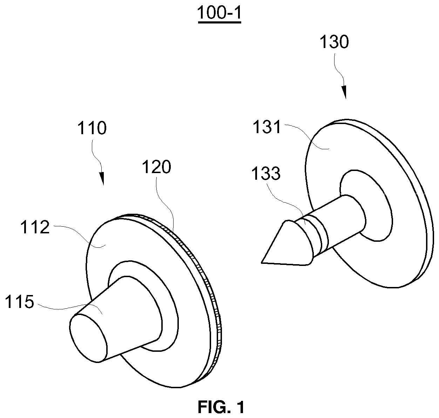

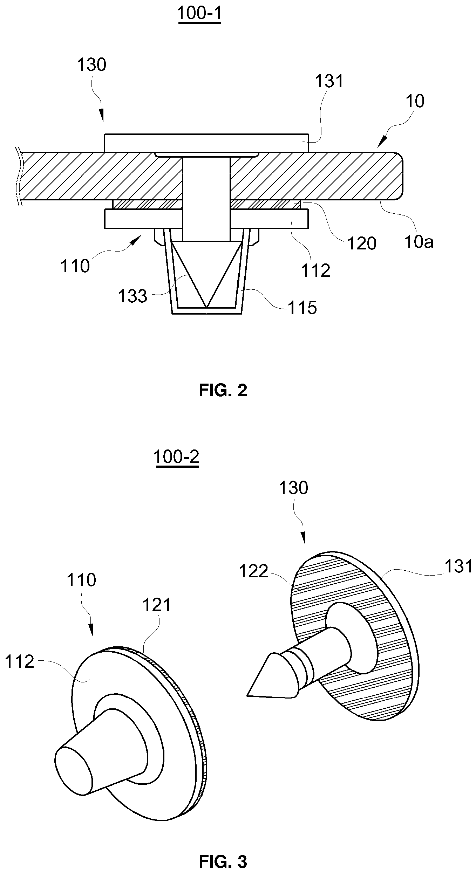

FIG. 1 is a perspective view schematically showing an appearance of a body temperature detecting marker 100-1 according to a first embodiment of the present disclosure; and FIG. 2 is a cross-sectional view schematically showing the appearance of the body temperature detecting marker 100-1 according to the first embodiment of the present disclosure.

Referring to FIG. 1 and FIG. 2, the body temperature detecting marker 100-1 of the present disclosure may be a detecting marker for checking body temperature of an animal 10. However, it is not limited to this use, and the body temperature detecting marker 100-1 may have a role of an ear tag. That is, the body temperature detecting marker 100-1 may have information on the animal 10 printed thereon. Information on the animal 10 may include at least one of birth date, gender, breed, and an individual identification number.

In addition, the body temperature detecting marker 100-1 of the present disclosure comprises a display member 110, a coupling member 130, and a color-changing member 120.

Specifically, the display member 110 may be configured so that a color change in the color-changing member 120 is visible. At least a portion of the display member 110 may be transparent or translucent. For example, the display member 110 may have a transmittance of visible light of 30% to 90%. The display member 110 may include a transparent or translucent material. The transparent material may be a plastic material. The transparent material may include at least one of polypropylene (PP), polyethylene (PE), thermoplastic polyurethane (TPU), rubber, and silicone. However, the transparent or translucent material is not necessarily limited to these examples, and any transparent and light material can be applied.

Furthermore, the coupling member 130 may be configured to be capable of being separated from or coupled to the display member 110. For example, the coupling member 130 may be configured to be capable of male and female coupling with a portion of the display member 110. For example, the coupling member 130 may include a tip 133 having an end portion having a horn shape. The display member 110 may include an accommodating part 115 having an internal space that is configured to accommodate at least a portion of the tip 133. The inner space of the accommodating part 115 may be formed as a space narrower than a size of the end portion of the tip 133 so that the end portion of the tip 133 can be fixed.

The color-changing member 120 may be located between the display member 110 and the coupling member 130. The color-changing member 120 may include a thermochromic pigment that is configured to discolor to a different color in a predetermined temperature range. Here, the thermochromic pigment can be applied as long as it is a known thermochromic pigment configured to discolor to a different color in a predetermined temperature range.

Alternatively, the color-changing member 120 may include a thermochromic pigment that is configured to become transparent in a predetermined temperature range. Here, the thermochromic pigment can be applied as long as it is a known thermochromic pigment configured to become transparent in a predetermined temperature range.

The predetermined temperature range may be 33 degrees Celsius to 43 degrees Celsius. However, the predetermined temperature range is not necessarily limited to the above numerical range, but may be a body temperature range that appears when the animal 10 on which the body temperature detecting marker 100-1 is installed becomes sick with a selected disease. For example, the selected disease may be swine fever, foot-and-mouth disease, avian influenza or the like.

The color-changing member 120 may be located to face an epidermis 10a of the animal 10. For example, as shown in FIG. 2, the color-changing member 120 may be located to face an ear epidermis 10a of the animal 10.

The color-changing member 120 may be configured so that, when the thermochromic pigment discolors to a different color in a predetermined temperature range, the discolored thermochromic pigment having a different color is visible from outside through the transparent display member 110. At this time, the color-changing member 120 may be configured so that, when the thermochromic pigment becomes transparent in a predetermined temperature range, the epidermis 10a of the animal 10 is visible from outside through the transparent display member 110.

Accordingly, the body temperature detecting marker 100-1 of the present disclosure comprises a color-changing member 120, which has at least a portion located between at least partially transparent display member 110 and the coupling member 130 and contains a thermochromic pigment that discolors to a different color or becomes transparent in a predetermined temperature range, thereby enabling easily checking of body temperature change of the animal 10 by naked eyes through a color change of the body temperature detecting marker 100-1. In addition, the present disclosure does not use a thermometer to make contact as in the prior art, and thus does not aggravate a disease of a sick animal or artificially transmit the disease to another animal. Moreover, the present disclosure is economical, as it is less expensive than conventional methods of checking body temperature of the animal 10 using a thermal imaging camera.

Furthermore, the body temperature detecting marker 100-1 of the present disclosure does not require temperature measurement through separate contact unlike a method of using a thermometer in the prior art and allows temperature of multiple animals 10 to be checked by naked eyes at once, thereby easily checking body temperature of multiple animals 10.

The body temperature detecting marker 100-1 of the present disclosure can check for body temperature of the animal 10 by naked eyes, isolate a sick animal 10 at an early stage, slow down or block spread of a disease to other animals 10, which increases productivity of livestock farms and reduces damage caused by the disease, thereby increasing economic profits. In addition, the body temperature detecting marker 100-1 of the present disclosure can speed up response to livestock infectious diseases such as foot-and-mouth disease and reduce damage caused by infectious diseases, thereby reducing disease management cost and reducing consumption of national budget.

The color-changing member 120 may be prepared by curing ink containing the thermochromic pigment, polymer resin, and solvent. Here, 'curing' means evaporating a solvent and crosslinking polymer resin. For example, the solvent may be a water-insoluble solvent. The solvent may be an organic solvent.

The color-changing member 120 may include a polymer resin that has been cured so that the ink cannot be erased by water or solvent. For example, a cured polymer resin may be an acrylic resin or a UV-based photocurable resin. That is, the color-changing member 120 may be prepared by applying a mixture of a thermochromic pigment, a solvent, and a curable polymer onto the display member 110 or the coupling member 130 and then curing the mixture.

The color-changing member 120 in the form of ink may be applied to the display member 110 or each of the display member 110 and the coupling member 130. The color-changing member 120 in a form of ink applied on the display member 110 or the coupling member 130 may be cured by at least one of solvent evaporation, heat curing, and photocuring.

For example, the color-changing member 120 in the form of ink may have a content of the thermochromic pigment of 1% by weight to 10% by weight, a content of the polymer resin (binder) of 20% by weight to 30% by weight, a content of the solvent of 10% by weight to 60% by weight, and a content of an additive of 0.5% by weight to 10% by weight. Here, the additive may be a preservative, a hardener or the like.

Accordingly, the body temperature detecting marker 100-1 of the present disclosure further contains a cured polymer resin, such that the color-changing member 120 is not dissolved by water or solvent, thereby effectively increasing durability.

In addition, the coupling member 130 may include a first support plate 131 and a tip 133. For example, as shown in FIG. 1, the first support plate 131 may have a circular plate shape.

The tip 133 may have a pillar shape protruding in one direction from one surface of the first support plate 131. The tip 133 may have a sharp end (i.e., a conical shape) so as to penetrate a portion of the animal 10.

Moreover, the display member 110 may include a second support plate 112 and an accommodating part 115. The second support plate 112 may have a circular plate shape. The display member 110 may have information on the animal 10 printed on one surface of the second support plate 112. Information on the animal 10 may include birth date, gender, breed, and an individual identification number.

The accommodating part 115 may have an internal space into which a portion of the tip 133 is inserted. For example, as shown in FIG. 1, the internal space may have a cylindrical shape.

Accordingly, the body temperature detecting marker 100-1 of the present disclosure is configured so that the tip 133 of the coupling member 130 and the accommodating part 115 of the display member 110 are coupled to each other, thereby having advantages that the body temperature detecting marker 100-1 is easy to be installed and that the body temperature detecting marker 100-1 can be well fixed to a portion of the animal 10.

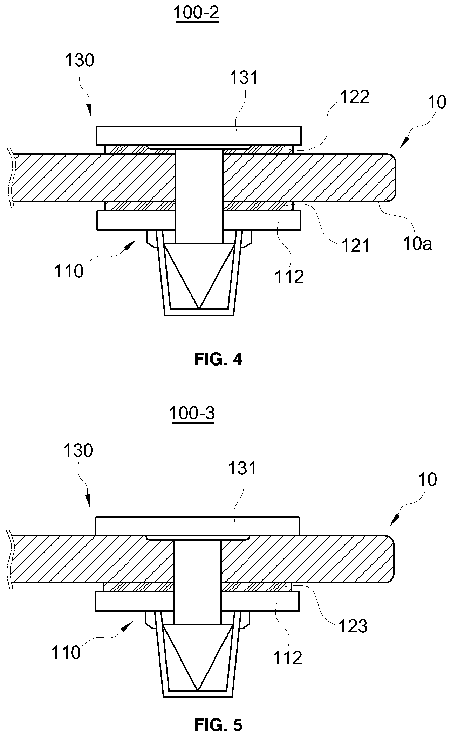

FIG. 3 is a perspective view schematically showing an appearance of a body temperature detecting marker 100-2 according to a second embodiment of the present disclosure; and FIG. 4 is a cross-sectional view schematically showing the appearance of the body temperature detecting marker 100-2 according to the second embodiment of the present disclosure.

Referring to FIG. 3 and FIG. 4, the body temperature detecting marker 100-2 according to the second embodiment of the present disclosure may comprise a first color-changing member 121 and a second color-changing member 122, compared to the body temperature detecting marker 100-1 according to the first embodiment. That is, as shown in FIG. 2, the body temperature detecting marker 100-1 according to the first embodiment is provided with one color-changing member 120 located to face the display member 110, whereas the body temperature detecting marker 100-2 according to the second embodiment is provided with a first color-changing member 121 located to face the epidermis on one side of an ear of the animal 10 and a second color-changing member 122 located to face the epidermis on the other side of the ear of the animal 10. In addition, the first color-changing member 121 may be located to face an inner side surface of the display member 110. The second color-changing member 122 may be located to face an inner side surface of the coupling member 130.

For example, the first color-changing member 121 may be formed by being applied on the second support plate 112. The second color-changing member 122 may be formed by being applied on the first support plate 131.

In addition, at least a portion of the coupling member 130 may be transparent. For example, the coupling member 130 may have a transmittance of visible light of 30% to 90%. The coupling member 130 may include a transparent material. The transparent material may be a plastic material. The transparent material may include at least one of polypropylene (PP), polyethylene (PE), thermoplastic polyurethane (TPU), rubber, and silicone.

Accordingly, the body temperature detecting marker 100-2 according to the second embodiment of the present disclosure comprises the first color-changing member 121 and the second color-changing member 122. If body temperature of the animal 10 rises due to a disease such as foot-and-mouth disease or the like, colors of each of the first color-changing member 121 and the second color-changing member 122 change or become transparent, by which the changed body temperature of the animal 10 can be checked through both the transparent display member 110 and the transparent coupling member 130, so that recognition rate for body temperature changes of the animal 10 by a user who breeds the animal 10 can be further increased.

FIG. 5 is a cross-sectional view schematically showing an appearance of a body temperature detecting marker 100-3 according to a third embodiment of the present disclosure.

Referring to FIG. 5, in a body temperature detecting marker 100-3 according to a third embodiment of the present disclosure, a color-changing member 123 may further contain a colored dye (not shown) that does not change according to temperature, unlike the body temperature detecting marker 100-1 according to the first embodiment. For example, the colored dye may have a different color from the thermochromic pigment. The colored dye can be a generally known dye, and any colored dye with a color different from the color or changed color of the thermochromic pigment can be applied. For example, the colored dye may be blue or red. For example, when the thermochromic pigment of the color-changing member 123 changes from black to transparent in a predetermined temperature range, a color (e.g., red) of the colored dye may be visible to outside through the transparent display member 110.

As shown in FIG. 5, the color-changing member 123 may be interposed between the display member 110 and the animal 10.

Accordingly, in the body temperature detecting marker 100-3 according to the third embodiment of the present disclosure, the color-changing member 120 further includes a colored dye that does not change according to temperature, such that when the color-changing member 120 becomes transparent in a predetermined temperature range, the color of the colored dye can be revealed to outside through the transparent display member 110, thereby enabling a breeder raising the animal 10 to check for body temperature change of the animal 10.

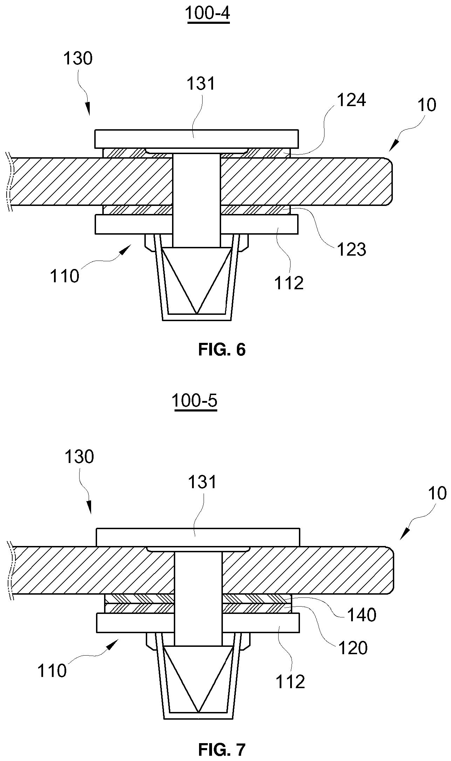

FIG. 6 is a cross-sectional view schematically showing an appearance of a body temperature detecting marker 100-4 according to a fourth embodiment of the present disclosure.

Referring to FIG. 6, in the body temperature detecting marker 100-4 according to the fourth embodiment of the present disclosure, the color-changing member 120 may include a third color-changing member 123 and a fourth color-changing member 124. Each of the third color-changing member 123 and the fourth color-changing member 124 may contain a colored dye. Accordingly, each of the third color-changing member 123 and the fourth color-changing member 124 changes to a different color which is a changed color of the thermochromic pigment at a predetermined temperature range, thereby representing a color that is mixed with the above-mentioned different color and the color of the colored dye.

Alternatively, at least a portion of the coupling member 130 is transparent. Therefore, in each of the third color-changing member 123 and the fourth color-changing member 124, as the thermochromic pigment becomes transparent in a predetermined temperature range, the color of the colored dye can be revealed to outside through each of the color-changing member 120 and the display member 110.

For example, the third color-changing member 123 may be located on an inner side surface of the second support plate 112 of the display member 110. For example, the third color-changing member 123 may be formed by being applied on the inner side surface of the second support plate 112 in a form of ink.

For example, the fourth color-changing member 124 may be located on an inner side surface of the first support plate 131 of the coupling member 130. For example, the fourth color-changing member 124 may be formed by being applied on the inner side surface of the first support plate 131 in a form of ink.

Accordingly, in the body temperature detecting marker 100-4 according to the fourth embodiment of the present disclosure, each of the third color-changing member 123 and the fourth color-changing member 124 further includes a colored dye that does not change according to temperature, such that when each of the third color-changing member 123 and the fourth color-changing member 124 becomes transparent in a predetermined temperature range, the color of the colored dye can be revealed to outside through the transparent display member 110, thereby enabling a breeder raising the animal 10 to check for a body temperature change of the animal 10.

FIG. 7 is a cross-sectional view schematically showing an appearance of a body temperature detecting marker 100-5 according to a fifth embodiment of the present disclosure.

Referring to FIG. 7, the body temperature detecting marker 100-5 according to a fifth embodiment of the present disclosure may further comprise a colored member 140, compared to the body temperature detecting marker 100-1 according to the first embodiment. For example, as shown in FIG. 7, the color-changing member 120 may be located between the epidermis of the animal 10 and the second support plate 112 of the display member 110. In addition, the colored member 140 may be configured to be interposed between the epidermis of the animal 10 and the color-changing member 120.

For example, the colored member may be located to be in close contact with an ear of the animal 10. The colored member 140 may be configured not to discolor in a predetermined temperature range. The predetermined temperature range may be 33 degrees Celsius to 43 degrees Celsius. For example, the color of the colored member may be blue, red, yellow, green or the like.

Accordingly, the body temperature detecting marker 100-5 according to the fifth embodiment of the present disclosure further comprises a colored member 140, such that when the color-changing member 120 becomes transparent in a predetermined temperature range, the color (e.g., red) of the colored member 140 can be revealed to outside through the transparent display member 110, thereby enabling a breeder raising the animal 10 to check for a body temperature change of the animal 10.

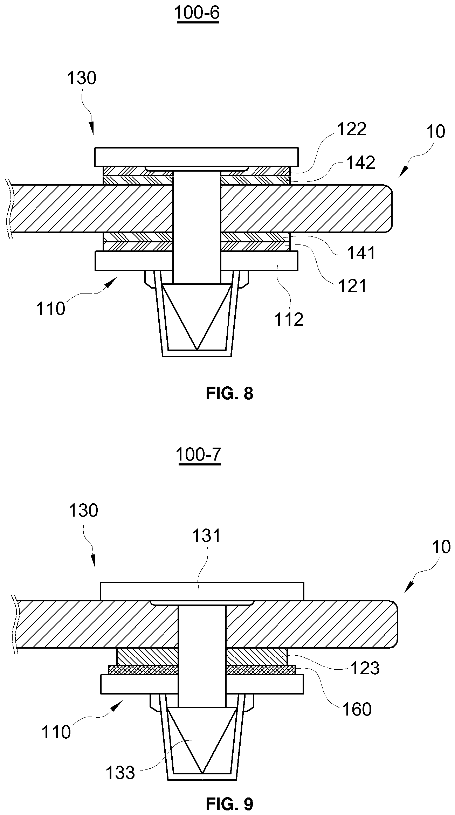

FIG. 8 is a cross-sectional view schematically showing an appearance of a body temperature detecting marker 100-6 according to a sixth embodiment of the present disclosure.

Referring to FIG. 8, the body temperature detecting marker 100-6 according to the sixth embodiment of the present disclosure may further comprise a first colored member 141 and a second colored member 142, compared to the body temperature detecting marker 100-1 according to the first embodiment. In addition, the body temperature detecting marker 100-6 may comprise a first color-changing member 121 and a second color-changing member 122. For example, as shown in FIG. 8, the first colored member 141 may be located between a portion of the animal 10 (e.g., ear) and the first color-changing member 121. For example, as shown in FIG. 8, the second colored member 142 may be located between a portion of the animal 10 (e.g., ear) and the second color-changing member 122.

The first colored member 141 and the second colored member 142 may be configured to be in close contact with one surface and the other surface of the epidermis of the animal 10, respectively. For example, each of the first colored member 141 and the second colored member 142 may be located to be in close contact with a front side and a rear side of an ear of the animal 10, respectively. Each of the first colored member 141 and the second colored member 142 may be configured not to discolor in a predetermined temperature range. The predetermined temperature range may be 33 degrees Celsius to 43 degrees Celsius. For example, the colors of the first colored member 141 and the second colored member 142 may be blue, red, yellow, green or the like.

Accordingly, the body temperature detecting marker 100-6 according to the sixth embodiment of the present disclosure further comprises a third color-changing member 123 and a fourth color-changing member 124 that do not change according to temperature, such that when each of the first color-changing member 121 and the second color-changing member 122 becomes transparent in a predetermined temperature range, the color of the colored dye can be revealed to outside through the transparent display member 110, thereby enabling a breeder raising the animal 10 to check for a body temperature change of the animal 10.

FIG. 9 is a cross-sectional view schematically showing an appearance of a body temperature detecting marker 100-7 according to a seventh embodiment of the present disclosure.

Referring to FIG. 9, the body temperature detecting marker 100-7 according to the seventh embodiment of the present disclosure may further comprise an elastic member 160 between the color-changing member 123 and the display member 110. The elastic member 160 may maintain the color-changing member 123 in close contact with an ear of the animal 10. The elastic member 160 may be formed of, for example, polyurethane foam, polyethylene foam, ethylene-vinyl acetate (EVA) foam, rubber form or the like.

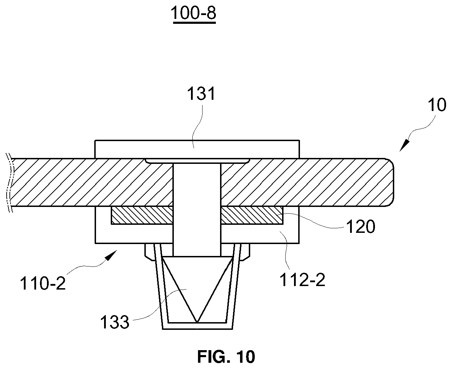

FIG. 10 is a cross-sectional view schematically showing an appearance of a body temperature detecting marker 100-8 according to an eighth embodiment of the present disclosure.

Referring to FIG. 10, the body temperature detecting marker 100-8 according to the eighth embodiment of the present disclosure may comprise a display member 110-2 having a structure in which an outer periphery protrudes toward an ear of the animal 10. That is, the outer periphery of a second support plate 112-2 of the display member 110-2 is formed to protrude toward the ear of the animal 10, thereby surrounding a boundary of the color-changing member 120. By this configuration of the second support plate 112-2 of the display member 110-2, airtightness of the color-changing member 120 can be improved.

Although the embodiments have been described by limited drawings as above, those skilled in the art may apply various technical modifications and variations based on the above. For example, even if the described technologies are performed in a different order from the described method, and/or components such as the described system, structure, device, and circuit are combined or mixed in a different form from the described method, or replaced by other components or equivalents, appropriate results can be achieved.

Accordingly, other implementations, other embodiments, and those equivalent to the claims fall within the scope of the claims described below.

## Claims

1. A body temperature detecting marker for checking body temperature of an animal, comprising:
a display member;
a coupling member configured to be capable of being separated from or coupled to the display member; and
a color-changing member located between the display member and the coupling member and containing a thermochromic pigment that discolors to a different color or becomes transparent in a predetermined temperature range,
wherein the display member is at least partially transparent or translucent so that a color change in the display member is visible;
the color-changing member further includes a cured polymer resin; and
the color-changing member is applied on the display member, or on the display member and the coupling member.

2. A body temperature detecting marker for checking body temperature of an animal, comprising:
a display member;
a coupling member configured to be capable of being separated from or coupled to the display member; and
a color-changing member located between the display member and the coupling member and containing a thermochromic pigment that discolors to a different color or becomes transparent in a predetermined temperature range,
wherein the display member is at least partially transparent or translucent so that a color change in the color-changing member is visible; and
the color-changing member includes:
a first color-changing member located to face one side of an ear of the animal; and
a second color-changing member located to face the other side of the ear of the animal.

3. The body temperature detecting marker according to Claim 1 or Claim 2,
wherein the predetermined temperature range is 33 degrees Celsius to 43 degrees Celsius.

4. The body temperature detecting marker according to Claim 1 or Claim 2,
wherein the color-changing member is located to face an epidermis of the animal.

5. The body temperature detecting marker according to Claim 1 or Claim 2,
wherein the color-changing member is prepared by curing ink containing the thermochromic pigment and a solvent.

6. The body temperature detecting marker according to Claim 1 or Claim 2,
wherein the coupling member includes:
a first support plate; and
a tip configured to protrude in one direction from one surface of the first support plate and penetrate a portion of the animal, and
the display member includes:
a second support plate; and
an accommodating part having a space into which a portion of the tip is inserted.

7. The body temperature detecting marker according to Claim 1 or Claim 2,
wherein the color-changing member further contains a colored dye that does not change according to temperature.

8. The body temperature detecting marker according to Claim 1 or Claim 2, further comprising,
a colored member that is interposed between the epidermis of the animal and the color-changing member, and does not discolor in a predetermined temperature range.
